Europäisches Patentamt

European Patent Office

Office européen des brevets

(19)

(11) Publication number: **0 133 731**
**B1**

(12) **EUROPEAN PATENT SPECIFICATION**

(45) Date of publication of patent specification: 29.07.87

(51) Int. Cl.⁴: **C 07 C 29/56, C 07 C 33/02**

(21) Application number: **84201139.7**

(22) Date of filing: **02.08.84**

(54) Epoxide isomerization process.

(30) Priority: **11.08.83 US 522130**

(43) Date of publication of application:
**06.03.85 Bulletin 85/10**

(45) Publication of the grant of the patent:
**29.07.87 Bulletin 87/31**

(84) Designated Contracting States:
**AT BE CH DE FR GB IT LI LU NL SE**

(56) References cited:
**DE-B-1 086 694**
**GB-A-1 166 557**

**CHEMICAL ABSTRACTS, vol. 70, no. 17, 28th
April 1969, page 379, column 2, abstract no.
78165x, Columbus, Ohio, US; E.H. ESCHINASI
"Aluminium alkoxide rearrangement of
epoxides. I. The synthesis of allylic alcohols
and glycol monoethers"**

(73) Proprietor: **SHELL INTERNATIONALE
RESEARCH MAATSCHAPPIJ B.V.
Carel van Bylandtlaan 30
NL-2596 HR Den Haag (NL)**

(72) Inventor: **Edwards, Charles Lee
1722 Ashford Hollow Lane
Houston Texas 77077 (US)**
Inventor: **Wilson, Stanley Edward
5207 Blueberry Hill Drive
Houston Texas 77084 (US)**

(74) Representative: **Hunter, Keith Roger Ian et al
4 York Road
London SE1 7NA (GB)**

Courier Press, Leamington Spa, England.

# 0 133 731

## Description

The present invention relates to the isomerization of certain epoxides to their corresponding allylic alcohols.

It is known from Eschinasi, E. H., *Israel Journal of Chemistry, 6,* pages 713—721 (1968) to use aluminum isopropoxide for the rearrangement of epoxides to allylic alcohols. However, this reaction has been found to be very slow, giving poor conversions and yields of the desired alcohols under practical reaction times. By contrast, it has been found that certain other metal alkoxides give much faster reactions with good conversion and very good selectivity to the desired alcohols.

The present invention accordingly relates to a process for the preparation of allylic alcohols by isomerizing an epoxide having at most two hydrogen atoms attached to the epoxy group in the presence of a metal alkoxide catalyst, characterised in that the catalyst is an alkoxide of a transition metal of Group IVB or VB of the Periodic Table of Elements (as set out in the Merck Index), and the reaction is carried out in the absence of a solvent.

The catalyst is a metal alkoxide in which the metal is a transition metal of Groups IVB or VB of the Periodic Table of Elements. As is well-known, transition metals are incorporated into the Periodic Table of Elements (after Mendeleev) by the division of groups 3—7 into sub-groups A and B. However, there is no established convention regarding the letter which designates the sub-group containing transition elements. To avoid any doubt, it should be noted that Groups IVB and VB are used herein to denote the relevant transition elements, following the Periodic Table format set out in the Merck Index, namely titanium, zirconium and hafnium (IVB) and vanadium, niobium and tantalum (VB). Preferably, the metal is titanium, zirconium or tantalum. Each alkyl moiety in the metal alkoxide can be any lower alkyl group, e.g. containing from 1 to 7 carbon atoms, but preferably each alkyl moiety contains 2, 3 or 4 carbon atoms. For example, the metal alkoxide is suitably titanium isopropoxide (Ti (O-iProp)$_4$), zirconium n-butoxide (Zr(O-nBu)$_4$), or tantalum ethoxide (Ta(O-Et)$_5$), including metal alkoxides coordinated with the alcohol used in making the alkoxide, e.g. Zr(O-nBu)$_4 \cdot$ nBuOH. Good results have been obtained with zirconium n-butoxide.

The metal alkoxide catalysts are known materials prepared by conventional methods of making metal alkoxides by reaction of the corresponding alkanol with the desired transition metal of Group IVB or VB described above.

Epoxides are characterised by the presence of an epoxy group, that is an oxygen atom attached to two adjacent carbon atoms. The epoxides used in the process of this invention are those in which at most two hydrogen atoms are attached to the epoxy group. Preferably, not more than one of the adjacent carbon atoms of the epoxy group forms part of a carbocyclic ring. These epoxides are readily prepared by conventional procedures known in the art for the epoxidation of aliphatic and cycloaliphatic mono- and non-conjugated polyolefins with e.g. hydrogen peroxide or hydrocarbyl hydroperoxides, preferably in the presence of various metal catalysts.

Examples of the olefins which can be used to prepare these epoxides include:—

(a) Aliphatic olefins, such as propylene, butenes, isobutene, hexenes, 4-methyl-2-pentene, etc;

(b) Cycloolefins, for example, cyclopentene, cyclohexene, cyclooctene, etc;

(c) Alkyl and alkenyl cycloolefins, for example, methylcyclohexene, methylcyclopentene, vinylcyclohexene;

(d) Compounds having a plurality of olefinic double bonds, unconjugated, for example, 1,5-cyclooctadiene, 1,5,9-cyclododecatriene, 1,4-cyclohexadiene, cyclohexadiene;

(e) Terpenes, for example, p-menthadienes such as terpinolene, terpinenes, etc.

The epoxide is suitably a carbocyclic olefinic epoxide in which no hydrogen atoms are attached to the epoxy group and one carbon of the epoxy group is part of the carbocyclic ring, thereby yielding an allylic alcohol in which the alcohol function is tertiary and is attached to the ring. Preferably, the epoxide is a terpenoid epoxides, i.e. one prepared by epoxidation of a terpene, including monocyclic, bicyclic and acylic terpenes, for example an epoxide prepared from epoxidation of a p-menthandiene, and, especially terpinolene epoxide.

The isomerization process of the invention is conducted in the absence of added solvent because conventional solvents, such as water, alcohols and hydrocarbons, may inhibit the reaction.

The isomerization process of the invention is preferably conducted by adding the metal alkoxide catalyst to the epoxide at ambient temperatures in an inert atmosphere, heating the reaction mixture to the desired temperature until the desired degree of conversion has been completed, cooling the reaction mixture and recovering the desired allylic alcohol product by conventional techniques, such as distillation, extraction and the like.

The rate of reaction varies somewhat with the catalyst concentration, which is preferably from 0.05 to 5 mole per cent per mole of epoxide, especially from 1 to 2 mole per cent of catalyst per mole of epoxide reactant.

The rate of conversion and selectivity to the desired product alcohol varies with the temperature of the reaction under normal pressures. A reaction temperature in the range of from 100°C to 170°C can be used, and a temperature in the range of from 135°C to 155°C, and especially from 130°C to 150°C, has been found to give high conversion in relatively short reaction times.

2

The present invention is advantageous as a process to isomerize epoxides in high conversion and very good selectivity with short reaction times.

The product allylic alcohols are known in the art and have application in organic synthesis, for examples as intermediates to the corresponding saturated alcohols, unsaturated halides, halo-substituted allyl alcohols, and the like. The alcohols derived by isomerization of epoxides made from terpenes can also have application in the perfumery industry. In particular, terpinene-4-ol is an important constituent of many so called "essential" oils and has similar utility.

The invention is illustrated in the following Examples. In each of these Examples the reaction was conducted by addition of a catalyst to a $N_2$-filled reaction vessel containing the terpinolene 4,7-epoxide at 25°C. The reaction vessel used was a four neck round bottom flask fitted with a $N_2$ inlet, reflux condensor, sampling tube and thermometer. The reflux condensor was connected to a $N_2$ bubbler which was capable of maintaining the unit at one atmosphere of $N_2$. The reaction mixture was heated to the desired reaction temperature at which time aliquots were withdrawn at specified periods and analyzed by GLC. A 3 mm×150 mm 10% SP-1000 on 100/120 Chromosorb WAW column was used for quantitative analysis.

Example 1

To 11 g of terpinolene 4,7-epoxide was added under nitrogen a single portion of 0.408 g of titanium isopropoxide at 25°C producing a clear pale gold-coloured solution. The reaction mixture was heated at 110°C—120°C for 18 hours at which time the solution was orange-coloured and contained water as an immiscible second liquid phase. After cooling to 25°C the mixture was analyzed by gas-liquid chromatography which showed that dipentene-4-ol had been produced at 75% conversion with 82% selectivity. Distillation of the crude reaction mixture gave a 51% yield of dipentene-4-ol, b.p. 73°C (0.4 mm).

Examples 2—7

Following procedures similar to those described in Example 1 above, terpinolene 4,7-epoxide was treated with various Group IVB and VB metal alkoxides to give dipenten-4-ol.

| Example | Metal alkoxide | Mole % | Time hours | Temp. °C | % Conversion of epoxide | Selectivity to dipenten-4-ol |
|---------|---------------|--------|-----------|---------|------------------------|------------------------------|
| 2 | Ta(OEt)₅ | 1 | 17 | 115 | 82 | 78 |
| 3 | Ta(OET)₅ | 2 | 1.5 | 150 | 86 | 73 |
| 4 | Zr(O-nBu)₄ | 1 | 14 | 125 | 90 | 70 |
| 5 | Zr(O-nBu)₄ | 2 | 2 | 150 | 88 | 83 |
| 6 | Zr(O-nBu)₄ · nBuOH | 2 | 12 | 130 | 74 | 85 |
| 7 | Ti(O-IProp)₄ | 2 | 15 | 110 | 66 | 79 |

**Claims**

1. A process for the preparation of allylic alcohols by isomerizing an epoxide having at most two hydrogen atoms attached to the epoxy group in the presence of a metal alkoxide catalyst, characterised in that the catalyst is an alkoxide of a transition metal of Group IVB or VB of the Periodic Table of Elements, and the reaction is carried out in the absence of a solvent.

2. A process as claimed in claim 1 wherein the transition metal is titanium, zirconium or tantalum.

3. A process as claimed in claim 1 or 2 wherein each alkyl moiety in the metal alkoxide contains 2, 3 or 4 carbon atoms.

4. A process as claimed in claim 3 wherein the metal alkoxide is titanium isopropoxide or zirconium n-butoxide.

5. A process as claimed in any one of claims 1 to 4 wherein the epoxide is a carboxylic olefinic epoxide in which no hydrogen atoms are attached to the epoxy group and one carbon atom of the epoxy group is part of the carbocyclic ring, thereby yielding an allylic alcohol in which the alcohol function is tertiary and is attached to the ring.

6. A process as claimed in claim 5 wherein the epoxide is a terpenoid epoxide.

7. A process as claimed in any one of the preceding claims wherein the reaction temperature is in the range of from 100°C to 170°C.

8. A process as claimed in any one of the preceding claims wherein the metal alkoxide catalyst is used in a concentration of from 0.05 to 5 mole percent per mole of epoxide.

# 0 133 731

**Patentansprüche**

1. Verfahren zur Herstellung von Allylalkoholen durch Isomerisieren eines Epoxids, das höchstens zwei Wasserstoffatome an die Epoxygruppe gebunden enthält, in Gegenwart eines Metallalkoxid-Katalysators, dadurch gekennzeichnet, daß der Katalysator ein Alkoxid eines Übergangsmetalles der Gruppe IVB oder VB des Periodensystems der Elemente ist und die Reaktion in Abwesenheit eines Lösungsmittels ausgeführt wird.

2. Verfahren nach Anspruch 1, worin das Übergangsmetall Titan, Zirkon oder Tantal ist.

3. Verfahren nach Anspruch 1 oder 2, worin jeder Alkylrest im Metallalkoxid 2, 3 oder 4 Kohlenstoffatome enthält.

4. Verfahren nach Anspruch 3, worin das Metallalkoxid Titanisopropoxid oder Zirkon-n-butoxid ist.

5. Verfahren nach einem der Ansprüche 1 bis 4, worin das Epoxid ein carbocyclisches olefinisches Epoxid ist, worin keine Wasserstoffatome an die Epoxygruppe gebunden sind und ein Kohlenstoffatom der Epoxygruppe Teil des carbocyclischen Ringes ist, wodurch ein Allylalkohol erhalten wird, worin die Alkoholfunktion tertiär ist und an den Ring gebunden ist.

6. Verfahren nach Anspruch 5, worin das Epoxid ein Terpenoidepoxid ist.

7. Verfahren nach einem der vorstehenden Ansprüche, worin die Reaktionstemperatur im Bereich von 100°C bis 170°C liegt.

8. Verfahren nach einem der vorstehenden Ansprüche, worin der Metallalkoxid-Katalysator in einer Konzentration von 0,05 bis 5 Mol-% je Mol Epoxid verwendet wird.

**Revendications**

1. Un procédé pour la préparation d'alcools allyliques par isomérisation d'un époxyde ayant au maximum deux atomes d'hydrogène attachés au groupe époxy en présence d'un catalyseur alcoolate de métal, caractérisé en ce que le catalyseur est un alcoolate d'un métal de transition du groupe IVB ou VB du tableau périodique des éléments et que la réaction est conduite en l'absence d'un solvant.

2. Un procédé selon la revendication 1, dans lequel le métal de transition est du titane, du zirconium ou du tantale.

3. Un procédé selon la revendication 1 ou 2, dans lequel chaque portion alcoyle dans l'alcoolate de métal contient 2, 3 ou 4 atomes de carbone.

4. Un procédé selon la revendication 3, dans lequel l'alcoolate de métal est de l'isopropylate de titane ou du n-butylate de zirconium.

5. Un procédé selon l'une quelconque des revendications 1 à 4, dans lequel l'époxyde est un époxyde oléfinique carbocyclique dans lequel il n'y a pas d'atomes d'hydrogène attachés au groupe époxy et un atome de carbone du groupe époxy fait partie du noyau carbocyclique, donnant ainsi un alcool allylique dans lequel la fonction alcool est tertiaire et est attachée au noyau.

6. Un procédé selon la revendication 5, dans lequel l'époxyde est un terpénoïde époxyde.

7. Un procédé selon l'une quelconque des revendications précédentes, dans lequel la température de réaction est comprise entre 100°C et 170°C.

8. Un procédé selon l'une quelconque des revendications précédentes, dans lequel le catalyseur alcoolate de métal est utilisé à une concentration de 0,05 à 5 moles pour cent par mole d'époxyde.